# EUROPÄISCHE PATENTSCHRIFT

(11) **EP 0 666 732 B1**
(45) Veröffentlichungstag und Bekanntmachung des Hinweises auf die Patenterteilung: **29.01.1997**
(21) Anmeldenummer: 93922925.8
(22) Anmeldetag: 09.10.1993
(51) Int. Cl.: A61K 7/32

(54) **DESODORIERENDE KOSMETISCHE ZUBEREITUNGEN MIT EINEM GEHALT AN DI- ODER TRIGLYCERINESTERN**
COSMETIC DEODORANT PREPARATIONS CONTAINING DI- OR TRIGLYCERIN ESTERS
PREPARATIONS COSMETIQUES DEODORANTES CONTENANT DES ESTERS DI- OU TRIGLYCERIQUES

(30) Priorität: 03.11.1992 DE 4237081
(43) Veröffentlichungstag der Anmeldung: 16.08.1995
(73) Patentinhaber: Beiersdorf Aktiengesellschaft, D-20245 Hamburg (DE); Solvay Fluor und Derivate GmbH, D-30173 Hannover (DE)
(72) Erfinder: DILLENBURG, Helmut, D-47495 Rheinberg (DE); JAKOBSON, Gerald, D-47495 Rheinberg (DE); KLEIN, Winfried, D-22297 Hamburg (DE); SIEMANOWSKI, Werner, D-47495 Rheinberg (DE); UHLIG, Karlheinz, D-47802 Krefeld (DE); WOLF, Florian, D-20251 Hamburg (DE)
(86) Internationale Anmeldenummer: EP9302767
(87) Internationale Veröffentlichungsnummer: WO9409753

(56) Entgegenhaltungen:
- EP-A- 0 379 658
- WO-A-90/10441
- DE-A- 3 930 193
- DE-C- 4 100 490
- US-A- 4 704 271

## Beschreibung

Die vorliegende Erfindung betrifft kosmetische Desodorantien. Solche Formulierungen dienen dazu, Körpergeruch zu beseitigen, der entsteht, wenn der an sich geruchlose frische Schweiß durch Mikroorganismen zersetzt wird. Den handelsüblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Zubereitungen, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Es wurde überraschend gefunden, und darin liegt die Lösung all dieser Aufgaben, daß die Verwendung von Monocarbonsäureestern des Di- und/oder Triglycerins zur Bekämpfung grampositiver, insbesondere coryneformer Bakterien oder zur Verhinderung deren Wachstums den Nachteilen des Standes der Technik abhilft.

Zwar ist bekannt, daß Fettsäureester des Glycerins (also des Monoglycerins) gewisse antimikrobielle Wirkung zeitigen. Bekannt ist ferner, Monoglycerinfettsäureester in desodorierenden Kosmetika einzusetzen, insbesondere das Glycerinmonolaurat. Dennoch bleibt die Wirkung dieser Monoglycerinester weit hinter der der erfindungsgemäßen Monocarbonsäureester zurück.

Die EP-A-0 379 658 beschreibt Wasch-, Reinigungs- und/oder Körperreinigungsmittel mit einem Gehalt an Oligoglycerinestern. Auch beschreibt die US-A 4,704,271 einen Antitranspirantstift mit einem Gehalt an bestimmten Polyglycerinen. In der Schritt WO 90/10441 wird die Verwendung bestimmter Triglycerinester gegen krankhafte Hauterscheinungen beschreiben.

Keine dieser Schriften konnte aber den Weg zur vorliegenden Erfindung aufzeigen.

Erfindungsgemäß liegen die Di- bzw. Triglycerineinheiten der erfindungsgemäßen Monocarbonsäureester als lineare, unverzweigte Moleküle, also über die jeweiligen OH-Gruppen in 1- bzw. 3-Stellung veretherte "Monoglycerinmoleküle" vor.

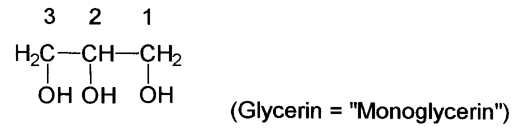

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Erfindungsgemäß sind die Monocarbonsäureester des Diglycerins vorzugsweise Monocarbonsäuremonoester und bevorzugt durch folgende Struktur gekennzeichnet (Substitutionspositionen angegeben): 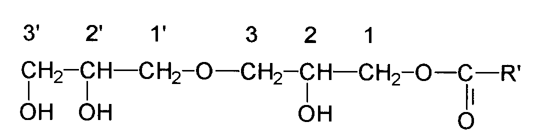 wobei R' einen Kohlenwasserstoffrest, vorteilhaft einen verzweigten oder unverzweigten Alkyl- oder Alkenylrest von 5 bis 17 C-Atomen darstellt.

Erfindungsgemäß sind die Monocarbonsäureester des Triglycerins vorzugsweise Monocarbonsäuremonoester und bevorzugt durch folgende Struktur gekennzeichnet (Substitutionspositionen angegeben): 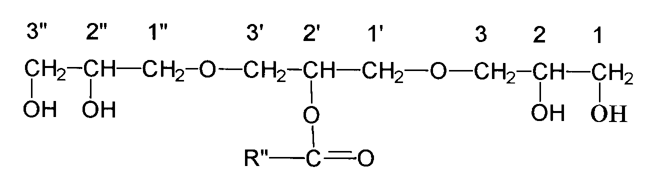 wobei R'' einen Kohlenwasserstoffrest, vorteilhaft einen verzweigten oder unverzweigten Alkyl- oder Alkenylrest von 5 bis 17 C-Atomen darstellt.

Die diesen Estern zugrundeliegenden Säuren sind die

| | | |
|---|---|---|
| Hexansäure | (Capronsäure) | (R' bzw. R'' = -C₅H₁₁), |
| Heptansäure | (Önanthsäure) | (R' bzw. R'' = -C₆H₁₃), |
| Octansäure | (Caprylsäure) | (R' bzw. R'' = -C₇H₁₅), |
| Nonansäure | (Pelargonsäure) | (R' bzw. R'' = -C₈H₁₇), |
| Decansäure | (Caprinsäure) | (R' bzw. R'' = -C₉H₁₉), |
| Undecansäure | | (R' bzw. R'' = -C₁₀H₂₁), |
| 10-Undecensäure | (Undecylensäure) | (R' bzw. R'' = -C₁₀H₁₉), |
| Dodecansäure | (Laurinsäure) | (R' bzw. R'' = -C₁₁H₂₃), |
| Tridecansäure | | (R' bzw. R'' = -C₁₂H₂₅), |
| Tetradecansäure | (Myristinsäure) | (R' bzw. R'' = -C₁₃H₂₇), |
| Pentadecansäure | | (R' bzw. R'' = -C₁₄H₂₉), |
| Hexadecansäure | (Palmitinsäure) | (R' bzw. R'' = -C₁₅H₃₁), |
| Heptadecansäure | (Margarinsäure) | (R' bzw. R'' = -C₁₆H₃₃), |
| Octadecansäure | (Stearinsäure) | (R' bzw. R'' = -C₁₇H₃₅). |

Besonders günstig werden R' und R'' gewählt aus der Gruppe der unverzweigten Alkylreste mit ungeraden C-Zahlen, insbesondere mit 9, 11 und 13 C-Atomen.

Im allgemeinen sind die Ester des Diglycerins denen des Triglycerins bevorzugt.

Ganz besonders günstig sind

| | | |
|---|---|---|
| Diglycerinmonocaprinat | (DMC) | R' = 9 |
| Triglycerinmonolaurat | (TML) | R'' = 11 |
| Diglycerinmonolaurat | (DML) | R' = 11 |
| Triglycerinmonomyristat | (TMM) | R'' = 13. |

Als erfindungsgemäß bevorzugter Monocarbonsäureester hat sich das Diglycerinmonocaprinat (DMC) erwiesen.

Erfindungsgemäß liegen die Monofettsäureester des Diglycerins bevorzugt in 1-Stellung, die erfindungsgemäßen Monofettsäureester des Triglycerins bevorzugt in 2'-Stellung verestert vor.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestern des Di- bzw. Triglycerins verwendet.

Insbesondere vorteilhaft sind solche Monocarbonsäureester, welche nach einem Verfahren erhältlich sind, wie es in der DE-OS 38 18 293 beschrieben wird.

Die Diglycerinester, welche sich durch zwei, und die Triglycerinester, welche sich durch drei Asymmetriezentren auszeichnen, sind in all ihren Konfigurationen erfindungsgemäß wirksam. Die Diglycerinester besitzen vier, die Triglycerinester acht Stereoisomere.

Bei den Diglycerinestern sind die 2- und die 2'-Position Asymmetriezentren. Erfindungsgemäß aktiv und gleichermaßen von Vorteil sind die 2S2'S-, die 2R2'S-, die 2S2'R- und die 2R2'R-Konfiguration.

Bei den Triglycerinestern sind die 2-, die 2' und die 2''-Position Asymmetriezentren. Erfindungsgemäß aktiv und gleichermaßen von Vorteil sind die 2S2'S2''S-, die 2R2'S2''S-, die 2S2'R2''S-, die 2R2'R2''S-, die 2S2'S2''R, die 2R2'S2''R-, die 2S2'R2''R- und die 2R2'R2''R-Konfiguration.

Es hat sich als günstig herausgestellt, racemische Gemische der Stereoisomeren zu verwenden.

Nach einer vorteilhaften Ausführungsform der vorliegenden Erfindung werden Gemische eines oder mehrerer Monocarbonsäureester des Diglycerins mit einem oder mehreren Monocarbonsäureestern des Triglycerins verwendet.

Nach einer weiteren vorteilhaften Ausführungsform der vorliegenden Erfindung werden ein oder mehrere Monocarbonsäureester des Diglycerins und/oder ein oder mehrere Monocarbonsäureester des Triglycerins in Kombination mit anderen in Kosmetika üblichen Wirkstoffen (Ersatzwirkstoffe), Hilfs-, Verschnitt- und/oder Zusatzstoffen eingesetzt.

Vorteilhaft liegen dann die Verschnittstoffe und/oder Ersatzwirkstoffe in einer Konzentration bis zu 50 Gew.-Teilen vor, bevorzugt bis zu 35 Gew.-Teilen, bezogen auf das 100 Gew.-Teile der Gesamtmenge, welche sich aus dem Monocarbonsäureester bzw. den Monocarbonsäureestern des Diglycerins und/oder des Triglycerins und diesen Ersatzwirkstoffen und/oder Verschnittstoffen zusammensetzt.

Nach einer weiteren vorteilhaften Ausführungsform der vorliegenden Erfindung werden ein oder mehrere Monocarbonsäureester des Diglycerins und/oder ein oder mehrere Monocarbonsäureester des Triglycerins in Kombination mit anderen desodorierend oder das Wachstum von Bakterien hemmend oder Bakterien abtötend wirkenden Stoffen eingesetzt.

Nach noch einer weiteren vorteilhaften Ausführungsform der vorliegenden Erfindung werden ein oder mehrere Monocarbonsäureester des Diglycerins und/oder ein oder mehrere Monocarbonsäureester des Triglycerins in Kombination mit Monocarbonsäureestern des Glycerins (also des "Monoglycerins") eingesetzt. Dabei übernehmen diese Monocarbonsäureester des Glycerins die Rolle der Verschnittstoffe und/oder Ersatzwirkstoffe und werden vorzugsweise in einer Konzentration bis zu 50 Gew.-Teilen, bevorzugt bis zu 35 Gew.-Teilen eingesetzt, bezogen auf 100 Gew.-Teile der Gesamtmenge, welche sich aus dem Monocarbonsäureester bzw. den Monocarbonsäureestern des Diglycerins und/oder des Triglycerins und diesen Monocarbonsäureestern des Glycerins zusammensetzt.

Solche Monocarbonsäureester des Glycerins sind günstig gekennzeichnet durch eine Struktur wie folgt: 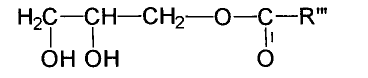 wobei R''' einen Kohlenwasserstoffrest, vorteilhaft einen verzweigten oder unverzweigten Alkyl- oder Alkenylrest von 5 bis 17 C-Atomen darstellt.

Die desodorierende Wirkung der erfindungsgemäß eingesetzten Ester beruht in erster Linie auf deren selektiver Toxizität für grampositive, insbesondere coryneforme Bakterien. Diese werden als die für die Zersetzung des apokrinen Schweißes hauptsächlich verantwortlichen Keime angesehen. Ferner sind die erfindungsgemäßen Ester gut wirksam gegen Staphylococcen.

Da die erfindungsgemäßen Ester zugleich völlig unschädlich für den Menschen und andere Warmblüter sind, sind sie in idealer Weise für die Verwendung in kosmetischen Desodorantien geeignet.

Erfindungsgemäß ist demnach auch die Verwendung von Monocarbonsäureestern des Di- und/oder Triglycerins als desodorierend wirkendes Prinzip für kosmetische Desodorantien.

Erfindungsgemäß ist ferner ein Verfahren zur Bekämpfung des durch mikrobielle Zersetzung apokrinen Schweißes hervorgerufenen menschlichen Körpergeruches, dadurch gekennzeichnet, daß eine wirksame Menge an Monocarbonsäureestern des Di- und/oder Triglycerins, welche gegebenenfalls in einem geeigneten kosmetischen Träger vorliegen können, auf die Haut aufgetragen wird.

Erfindungsgemäß ist schließlich auch die Verwendung von Monocarbonsäureestern, insbesondere von Monocarbonsäuremonoestern, des Di- und/oder Triglycerins zur Bekämpfung grampositiver, insbesondere coryneformer Bakterien, beziehungsweise die Verwendung von Monocarbonsäureestern des Di- und/oder Triglycerins zur Verhinderung des Wachstums grampositiver, insbesondere coryneformer Bakterien.

Entsprechend der erfindungsgemäßen Verwendung sind die Desodorantien besonders vorteilhaft dadurch gekennzeichnet, daß der oder die Monocarbonsäureester des Di- und/oder Triglycerins in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Entsprechend der erfindungsgemäßen Verwendung können die kosmetischen Desodorantien in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, als Deo-Stifte (Deo-Sticks) und in Form von aus normalen Flaschen und Behältern auftragbaren W/O- oder O/W-Emulsionen, z.B. Crèmes oder Lotionen. Weiterhin können die kosmetischen Desodorantien vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Als übliche kosmetische Trägerstoffe zur Herstellung der desodorierenden Zubereitungen gemäß der erfindungsgemäßen Verwendung können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für aus Aerosolbehältern versprühbare kosmetische Desodorantien gemäß der erfindungsgemäßen Verwendung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der kosmetischen Desodorantien gemäß der erfindungsgemäßen Verwendung, welche vorteilhaft als flüssige Zubereitungen mittels einer Roll-on-Vorrichtung auf die gewünschten Hautbereiche aufgetragen werden sollen, und die in den Zubereitungen in geringer Menge, z.B. 2 bis 5 Gewichts.-%, bezogen auf die Gesamt-Zusammensetzung, verwendet werden können, haben sich nichtionogene Typen, wie Polyoxyethylen-Fettalkoholether, z.B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den desodorierenden kosmetischen Zubereitungen gemäß der erfindungsgemäßen Verwendung, deren pH-Wert vorzugsweise z.B. durch übliche Puffergemische auf 4,0 bis 9,0 insbesondere 5,0 bis 6,5, eingestellt wird, Parfüm, Farbstoffe, Antioxidantien (z.B. _-Tocopherol und seine Derivate oder Butylhydroxytoluol (BHT = 2,6-Di-Tert.-butyl-4-methylphenol) in Mengen von 0,01 bis 0,03 %, bezogen auf die Gesamtzusammensetzung), Suspendiermittel, Puffergemische oder andere übliche kosmetische Grundstoffe beigemischt werden.

Vorteilhaft wird der pH-Wert der Formulierungen gemäß der erfindungsgemäßen Verwendung im schwach sauren bis schwach alkalischen Bereich eingestellt, bevorzugt von 4,0 - 9,0 , besonders bevorzugt von 5,0 - 6,5.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Zur Parfümierung sind gegebenenfalls auch solche Substanzen und Parfümöle geeignet, die stabil sind, die Haut nicht reizen und bereits als solche antibakterielle oder bakteriostatische Eigenschaften besitzen.

Die Herstellung der kosmetischen Zubereitungen erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von kosmetischen Formulierungen zu beachten, die dem Fachmann geläufig sind.

Sollen die Di- und/oder Triglycerinester gemäß der erfindungsgemäßen Verwendung in Pudersprays eingearbeitet werden, so können die Suspensionsgrundlagen dafür vorteilhaft gewählt werden aus der Gruppe

Kieselsäuregele (z.B. solche die unter dem Handelsnamen Aerosil^{R} erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung.

### Beispiel 1

### Aerosolspray I

| (a) Flüssige Phase | Gew.-% |
|---|---|
| Octyldodecanol | 0,50 |
| DMC | 0,50 |
| Parfüm | q.s. |
| Ethylalkohol | ad 100,00 |
| (b) Die unter (a) erhaltene flüssige Phase wird zusammen mit einem Propan/Butan 2,7 - Gemisch im Verhältnis 39 : 61 in Aerosolbehälter abgefüllt. | |

### Beispiel 2

### Aerosolspray II

| (a) Flüssige Phase | Gew.-% |
|---|---|
| Octyldodecanol | 0,50 |
| DMC | 0,20 |
| Parfüm | q.s. |
| Isopropylalkohol | ad 100,00 |
| (b) Die unter (a) erhaltene flüssige Phase wird zusammen mit einem Propan/Butan 2,7 - Gemisch im Verhältnis 39 : 61 in Aerosolbehälter abgefüllt. | |

### Beispiel 3

### Pumpspray I

| (a) | Gew.-% |
|---|---|
| Ethylalkohol | 60,00 |
| Glycerin | 1,00 |
| PEG-40-Hydriertes Ricinusöl | 2,00 |
| DMC | 0,50 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 4

### Roll on - Gel I

| (a) | Gew.-% |
|---|---|
| 1,3-Butylenglycol | 2,00 |
| Hydroxyethylcellulose | 0,50 |
| (z.B. Tylose 4000, Hoechst) | |

| (b) | |
|---|---|
| Ethylalkohol | 60,00 |
| PEG-40-Hydriertes Ricinusöl | 2,00 |
| DMC | 0,30 |
| Parfum | q.s. |

| (c) | |
|---|---|
| Wasser | ad 100,00 |

Die unter (a) genannten Bestandteile werden dispergiert, Wasser (c) wird zugegeben, bei Raumtemperatur quellen gelassen, eine Lösung der unter (b) genannten Bestandteile wird nach ca. 15 Minuten zugegeben. Die entstanden Mischung wird homogenisiert und kann abgefüllt werden.

### Beispiel 5

### Wachsstift I

| | Gew.-% |
|---|---|
| Hydriertes Ricinusöl | 5,00 |
| Bienenwachs | 6,00 |
| Ceresin (Hart-Ozokerit) | 30,00 |
| C₁₂₋₁₅-Alkohol-Benzoate | 17,00 |
| DMC | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 6

### Roll on - Emulsion I

| (a) | Gew.-% |
|---|---|
| Tricetearethphosphat | 0,30 |
| Octyldodecanol | 2,00 |
| C₁₂₋₁₅-Alkohol-Benzoate | 2,00 |
| DMC | 0,50 |
| C₁₀₋₃₀-Alkylacrylate | 0,15 |

| (b) | |
|---|---|
| Ethylalkohol | 10,00 |
| Parfum | q.s. |

| (c) | |
|---|---|
| NaOH | 0,05 |
| Wasser | ad 100,00 |

Die unter (a) und (c) genannten Bestandteile werden jeweils unter Rühren auf 75° C erwärmt. Sodann werden die Bestandteile (a) zu (c) gegeben. Die Mischung wird auf 35° C abgekühlt. Aus den Bestandteilen (b) wird eine Lösung hergestellt, welche auf 35° C erwärmt wird und unter Rühren zur Mischung aus (a) und (c) gegeben wird.

### Beispiel 7

### Aerosolspray IV

| (a) Flüssige Phase | Gew.-% |
|---|---|
| Octyldodecanol | 0,50 |
| TML | 0,20 |
| Parfüm | q.s. |
| Isopropylalkohol | ad 100,00 |
| (b) Die unter (a) erhaltene flüssige Phase wird zusammen mit einem Propan/Butan 2,7 - Gemisch im Verhältnis 39 : 61 in Aerosolbehälter abgefüllt. | |

### Beispiel 8

### Pumpspray II

| (a) | Gew.-% |
|---|---|
| Ethylalkohol | 60,00 |
| Glycerin | 1,00 |
| PEG-40-Hydriertes Ricinusöl | 2,00 |
| TML | 0,50 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 9

### Roll on - Gel II

| (a) | Gew.-% |
|---|---|
| 1,3-Butylenglycol | 2,00 |
| Hydroxyethylcellulose (z.B. Tylose 4000, Hoechst) | 0,50 |

| (b) | |
|---|---|
| Ethylalkohol | 60,00 |
| PEG-40-Hydriertes Ricinusöl | 2,00 |
| TML | 0,30 |
| Parfum | q.s. |

| (c) | |
|---|---|
| Wasser | ad 100,00 |

Die unter (a) genannten Bestandteile werden dispergiert, Wasser (c) wird zugegeben, bei Raumtemperatur quellen gelassen, eine Lösung der unter (b) genannten Bestandteile wird nach ca. 15 Minuten zugegeben. Die entstanden Mischung wird homogenisiert und kann abgefüllt werden.

### Beispiel 10

### Wachsstift II

| | Gew.-% |
|---|---|
| Hydriertes Ricinusöl | 5,00 |
| Bienenwachs | 6,00 |
| Ceresin (Hart-Ozokerit) | 30,00 |
| C₁₂₋₁₅-Alkohol-Benzoate | 17,00 |
| TML | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 11

### Roll on - Emulsion II

| (a) | Gew.-% |
|---|---|
| Tricetearethphosphat | 0,30 |
| Octyldodecanol | 2,00 |
| C₁₂₋₁₅-Alkohol-Benzoate | 2,00 |
| TML | 0,50 |
| C₁₀₋₃₀-Alkylacrylate | 0,15 |

| (b) | |
|---|---|
| Ethylalkohol | 10,00 |
| Parfum | q.s. |

| (c) | |
|---|---|
| NaOH | 0,05 |
| Wasser | ad 100,00 |

Die unter (a) und (c) genannten Bestandteile werden jeweils unter Rühren auf 75° C erwärmt. Sodann werden die Bestandteile (a) zu (c) gegeben. Die Mischung wird auf 35° C abgekühlt. Aus den Bestandteilen (b) wird eine Lösung hergestellt, welche auf 35° C erwärmt wird und unter Rühren zur Mischung aus (a) und (c) gegeben wird.

### Beispiel 12

### Pumpspray III

| (a) | Gew.-% |
|---|---|
| Ethylalkohol | 60,00 |
| Glycerin | 1,00 |
| PEG-40-Hydriertes Ricinusöl | 2,00 |
| DML | 0,50 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 13

### Roll on - Gel III

| (a) | Gew.-% |
|---|---|
| 1,3-Butylenglycol | 2,00 |
| Hydroxyethylcellulose (z.B. Tylose 4000, Hoechst) | 0,50 |

| (b) | |
|---|---|
| Ethylalkohol | 60,00 |
| PEG-40-Hydriertes Ricinusöl | 2,00 |
| DML | 0,30 |
| Parfum | q.s. |

| (c) | |
|---|---|
| Wasser | ad 100,00 |

Die unter (a) genannten Bestandteile werden dispergiert, Wasser (c) wird zugegeben, bei Raumtemperatur quellen gelassen, eine Lösung der unter (b) genannten Bestandteile wird nach ca. 15 Minuten zugegeben. Die entstanden Mischung wird homogenisiert und kann abgefüllt werden.

### Beispiel 14

### Wachsstift III

| | Gew.-% |
|---|---|
| Hydriertes Ricinusöl | 5,00 |
| Bienenwachs | 6,00 |
| Ceresin (Hart-Ozokerit) | 30,00 |
| C₁₂₋₁₅-Alkohol-Benzoate | 17,00 |
| DML | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 15

### Roll on - Emulsion III

| (a) | Gew.-% |
|---|---|
| Tricetearethphosphat | 0,30 |
| Octyldodecanol | 2,00 |
| C₁₂₋₁₅-Alkohol-Benzoate | 2,00 |
| DML | 0,50 |
| C₁₀₋₃₀-Alkylacrylate | 0,15 |

| (b) | |
|---|---|
| Ethylalkohol | 10,00 |
| Parfum | q.s. |

| (c) | |
|---|---|
| NaOH | 0,05 |
| Wasser | ad 100,00 |

Die unter (a) und (c) genannten Bestandteile werden jeweils unter Rühren auf 75° C erwärmt. Sodann werden die Bestandteile (a) zu (c) gegeben. Die Mischung wird auf 35° C abgekühlt. Aus den Bestandteilen (b) wird eine Lösung hergestellt, welche auf 35° C erwärmt wird und unter Rühren zur Mischung aus (a) und (c) gegeben wird.

### Beispiel 16

### Pumpspray IV

| (a) | Gew.-% |
|---|---|
| Ethylalkohol | 60,00 |
| Glycerin | 1,00 |
| PEG-40-Hydriertes Ricinusöl | 2,00 |
| DMC | 0,20 |
| TML | 0,30 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

Der nachfolgend dargestellte Versuch soll die überlegenheit der erfindungsgemäßen Monocarbonsäureester belegen.

### Versuch:

- Testorganismus:: Corynebacterium xerosis
- Eingesetzte Lösungen::
- AC-Medium:: 37 g Brain Heart Infusion, 5 g Glucose, 1 ml Tween 80 ad 1 l H₂O
- Testsubstanz 1:: Glycerinmonolaurat (GML) (Vergleichssubstanz gemäß dem Stande der Technik)
- Testsubstanz 2:: Diglycerinmonocaprinat (DMC)
- Kontrolle:: AC-Medium ohne Wirkstoff
- Testlösungen:: 4 mMol der jeweiligen Testsubstanz in AC-Medium

50 ml AC-Medium wurden mit 0,2 ml einer frischen Übernachtkultur des Testorganismus Corynebacterium xerosis (C. xerosis) beimpft. Das Kulturgefäß wurde bei 30° C mit 250 Upm geschüttelt, bis eine Zelldichte von 10⁵ bis 10⁶ Zellen/ml erreicht war.

Die Testsubstanzen wurden eingewogen, mit 5 ml AC-Medium versetzt und durch ca. 10-minütiges Erwärmen auf 60° C gelöst.

Anschließend wurden 5 ml-Aliquots der Bakteriensuspension mit den so gewonnenen Testlösungen versetzt und unter Beibehaltung der Wachstumsbedingungen zu verschiedenen Zeitpunkten Proben entnommen und deren Bakterientiter durch Ausplattieren von Verdünnungen auf Nährmedien bestimmt.

Im Diagramm sind die Wachstumskurven für die vorabbeschriebenen Versuchsansätze aufgezeigt. Es bedeuten
- - o - :: Testlösung 1
- - * - :: Testlösung 2
- - + - :: Kontrolle (Wirkstoff-freies AC-Medium)

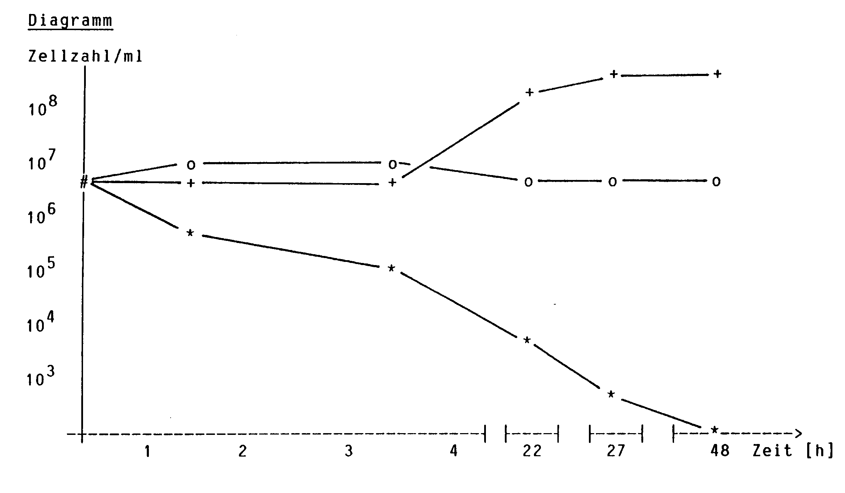

## Patentansprüche

1. Verwendung von Monocarbonsäureestern des Di- und/oder Triglycerins als desodorierend wirkendes Prinzip für kosmetische Desodorantien.

2. Verwendung von Monocarbonsäureestern des Di-und/oder Triglycerins zur Bekämpfung coryneformer Bakterien, mit Ausnahme der Propionibakterien, oder zur Verhinderung deren Wachstums.

3. Verfahren zur Bekämpfung des durch mikrobielle Zersetzung apokrinen Schweißes hervorgerufenen menschlichen Körpergeruches, dadurch gekennzeichnet, daß eine wirksame Menge an Monocarbonsäureestern des Di- und/oder Triglycerins, welche gegebenenfalls in einem geeigneten kosmetischen Träger vorliegen können, auf die Haut aufgetragen wird.

4. Verwendung oder Verfahren nach einem der vorhergehenden Ansprüche, daß das Diglycerin in 1-Stellung und/oder das Triglycerin In 2'-Stellung mit Carbonsäuren verestert vorliegt.

5. Verwendung oder Verfahren nach einem der vorhergehenden Ansprüche, dadurch gekennzeichnet, daß die Monocarbonsäureester des Diglycerins Monocarbonsäuremonoester darstellen und durch folgende Strukturen gekennzeichnet sind: 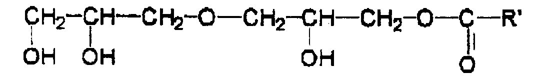 wobei R' einen Kohlenwasserstoffrest, vorteilhaft einen verzweigten oder unverzweigten Alkyl- oder Alkenylrest von 5 bis 17 C-Atomen darstellt.

6. Verwendung oder Verfahren nach einem der vorhergehenden Ansprüche, dadurch gekennzeichnet, daß die Monocarbonsäureester des Triglycerins Monocarbonsäuremonoester darstellen und durch folgende Strukturen gekennzeichnet sind: 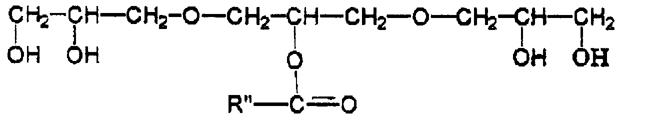 wobei R'' einen Kohlenwasserstoffrest, vorteilhaft einen verzweigten oder unverzweigten Alkyl- oder Alkenylrest von 5 bis 17 C-Atomen darstellt.

7. Verwendung oder Verfahren nach Anspruch 5 oder 6, dadurch gekennzeichnet, daß R' und/oder R'' gewählt werden aus der Gruppe der unverzweigten Alkylreste mit ungeraden C-Zahlen, insbesondere mit 9, 11 und 13 C-Atomen, bevorzugt aus der Gruppe
| | |
|---|---|
| Diglycerinmonocaprinat (DMC) | R' = 9 |
| Triglycerinmonolaurat (TML) | R'' = 11 |
| Diglycerinmonolaurat (DML) | R' = 11 |
| Triglycerinmonomyristat (TMM) | R'' = 13, |
besonders bevorzugt Diglycerinmonocaprinat, gewählt wird.

8. Verwendung oder Verfahren nach einem der vorhergehenden Ansprüche, dadurch gekennzeichnet, daß der oder die Monocarbonsäureester des Di- und/oder Triglycerins in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

## Claims

1. Use of monocarboxylic acid esters of di- and/or triglycerol as the deodorizing principle for cosmetic deodorants.

2. Use of monocarboxylic acid esters of di- and/or triglycerol for combating coryneform bacteria, with the exception of propionibacteria, or for preventing the growth thereof.

3. Process for combating human body odour caused by microbial decomposition of apocrine perspiration, characterized in that an active amount of monocarboxylic acid eaters of di- and/or triglycerol, which can be present in a suitable cosmetic carrier if appropriate, is applied to the skin.

4. Use or process according to one of the preceding claims, [lacuna] in that the diglycerol is esterified with carboxylic acids in the 1-position and/or the triglycerol is esterified with carboxylic acids in the 2'-position.

5. Use or process according to one of the preceding claims, characterized in that the monocarboxylic acid eaters of diglycerol are monocarboxylic acid monoesters and are characterized by the following structures: 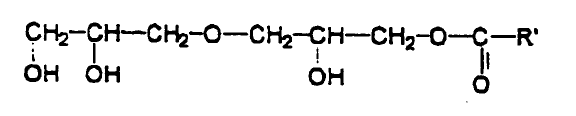 wherein R' is a hydrocarbon radical, advantageously a branched or unbranched alkyl or alkenyl radical having 5 to 17 C atoms.

6. Use or process according to one of the preceding claims, characterized in that the monocarboxylic acid eaters of triglycerol are monocarboxylic acid monoesters and are characterized by the following structures: 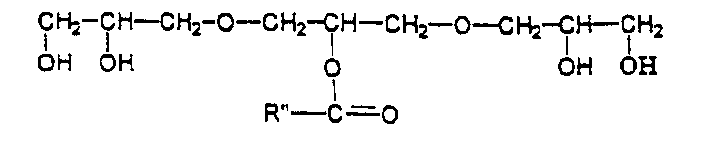 wherein R'' is a hydrocarbon radical, advantageously a branched or unbranched alkyl or alkenyl radical having 5 to 17 C atoms.

7. Use or process according to Claim 5 or 6, characterized in that R' and/or R'' are chosen from the group consisting of unbranched alkyl radicals having odd C numbers, in particular having 9, 11 and 13 C atoms, preferably from the group consisting of
| | | |
|---|---|---|
| diglycerol monocaprate | (DMC) | R' = 9 |
| triglycerol monolaurate | (TML) | R'' = 11 |
| diglycerol monolaurate | (DML) | R' = 11 |
| triglycerol monomyristate | (TMM) | R'' = 13, |
particularly preferably diglycerol monocaprate.

8. Use or process according to one of the preceding claims, characterized in that the monocarboxylic acid ester or esters of di- and/or triglycerol is or are present in concentrations of 0.01 - 10.00% by weight, preferably 0.05 - 5.00% by weight, particularly preferably 0.1 - 3.00% by weight, in each case based on the total weight of the composition.

## Revendications

1. Utilisation d'esters d'acides monocarboxyliques du diglycérol et/ou du triglycérol, en tant que principe à action déodorante pour des déodorants cosmétiques.

2. Utilisation d'esters d'acides monocarboxyliques du diglycérol et/ou du triglycérol pour lutter contre des bactéries corynéformes, à l'exception des propioni-bactéries, ou pour empêcher leur croissance.

3. Procédé pour combattre l'odeur corporelle humaine provoquée par la décomposition microbienne de la sueur apocrine, caractérisé en ce que l'on applique sur la peau une quantité efficace d'esters d'acides monocarboxyliques du diglycérol et/ou du triglycérol, qui peuvent éventuellement se trouver dans un véhicule cosmétique approprié.

4. Utilisation ou procédé selon l'une des revendications précédentes, caractérisé(e) en ce que le diglycérol se trouve à l'état estérifié en position 1 et/ou le triglycérol se trouve à l'état estérifié en position 2', par des acides carboxyliques.

5. Utilisation ou procédé selon l'une des revendications précédentes, caractérisé(e) en ce que les esters d'acides monocarboxyliques du diglycérol représentent des monoesters d'acides monocarboxyliques et sont caractérisés par les structures suivantes: 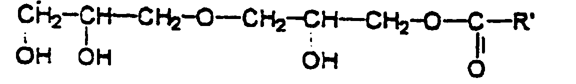 dans lesquelles R' représente un radical hydrocarboné, avantageusement un radical alkyle ou alcényle ramifié ou non ramifié, ayant de 5 à 17 atomes de carbone.

6. Utilisation ou procédé selon l'une des revendications précédentes, caractérisé(e) en ce que les esters d'acides monocarboxyliques du triglycérol représentent des monoesters d'acides monocarboxyliques et sont caractérisés par les structures suivantes: 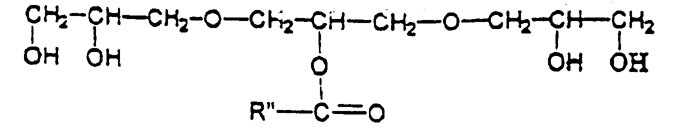 dans lesquelles R'' représente un radical hydrocarboné, avantageusement un radical alkyle ou alcényle ramifié ou non ramifié, ayant de 5 à 17 atomes de carbone.

7. Utilisation ou procédé selon la revendication 5 ou 6, caractérisé(e) en ce que R' et/ou R'' sont choisis dans le groupe constitué par les radicaux alkyle non ramifiés à nombres impairs d'atomes de carbone, en particulier à 9, 11 et 13 atomes de carbone, de préférence dans le groupe constitué par
| | |
|---|---|
| le monocaprate de diglycérol (DMC) | R' = 9, |
| le monolaurate de triglycérol (TML) | R'' = 11, |
| le monolaurate de diglycérol (DML) | R' = 11, |
| le monomyristate de triglycérol (TMM) | R'' = 13, |
et de facon particulièrement préférée on choisit le monocaprate de diglycérol.

8. Utilisation ou procédé selon l'une des revendications précédentes, caractérisé(e) en ce que le ou les esters d'acides monocarboxyliques du diglycérol et/ou du triglycérol se trouve(nt) à des concentrations de 0,01-10,00 % en poids, de préférence de 0,05-5,00 % en poids, de façon particulièrement préférée de 0,1-3,00 % en poids, dans chaque cas par rapport au poids total de la composition.
